# DEMANDE DE BREVET EUROPEEN

(11) **EP 1 175 888 A2**
(43) Date de publication de la demande: **30.01.2002**
(21) Numéro de dépôt: 01401865.9
(22) Date de dépôt: 11.07.2001
(51) Int. Cl.: A61K 7/06, A61K 7/48

(54) **Compositions à application topique comprenant des hydroxystilbènes glucosyles et utilisations**

(30) Priorité: 28.07.2000 FR 0010008
(71) Demandeur: L'OREAL, 75008 Paris (FR)
(72) Inventeur: Pruche, Françis, 60300 Senlis (FR); Bernard, Dominique, 75015 Paris (FR); Mehul, Bruno, 92800 Villejuif (FR)
(74) Mandataire: Desaix, Anne

(57) **Abrégé**

La présente invention est relative à des compositions appropriées pour l'application topique comprenant des hydroxystilbènes glucosylés. Elle porte aussi sur un procédé de libération progressive des hydroxystilbènes au niveau du stratum corneum, par l'application d'une composition comprenant des hydroxystilbènes glucosylés comme précurseur. Elle porte enfin sur l'utilisation des hydroxystilbènes glucosylés pour lutter contre les signes du vieillissement cutané et du follicule pileux, pour améliorer l'éclat du teint, pour lisser la peau du visage, pour traiter ou prévenir les rides et les ridules de la peau ou pour stimuler le processus de renouvellement épidermique.

## Description

La présente invention est relative à l'utilisation des hydroxystilbènes glucosylés et en particulier du resvératrol glucosylé comme précurseur d'hydroxystilbènes, à des compositions à application topique comprenant des hydroxystilbènes glucosylés et à un procédé de libération progressive des hydroxystilbènes au niveau du stratum corneum, par l'application topique d'une composition comprenant des hydroxystilbènes glucosylés.

Les stilbènes et stilbènes glucosylés sont produits chez les plantes, essentiellement chez les spermatophytes, et font partie de la classe des molécules antibiotiques connues sous le nom de phytoalexines. Parmi celles-ci, un exemple bien documenté est le resvératrol ou 3, 4', 5-trihydroxystilbène.

Le resvératrol existe naturellement dans de nombreuses plantes et fruits sous forme simple (trihydroxystilbène) ou glucosylée (piceid, polydatine ou 4',5-dihydroxystilbène-3-O-β-mono-D-glucoside, par exemple). On trouve les deux formes, simple et glucosylée, en particulier dans la peau du raisin (Vrhovsek *et al*., Am. J. Enol. Vitic., vol. 48, n° 2, 1997) ou encore dans des sumageants de cultures *in vitro* de *vitis vinifera* (Teguo *et al.*, J. Nat. Prod., 61, 655-657, 1998). En présence de β-glucosidases, le resvératrol est libéré. Cette réaction se produit naturellement chez la plante, par exemple, au niveau de la peau du raisin. Au cours de la fermentation des vins rouges (fermentation alcoolique), cette réaction est réalisée par les glycosidases des levures, mais elle n'est pas totale et il reste une proportion importante de dérivés glucosylés. La forme glucosylée est présente en quantité variable selon les vins, certaines variétés de Pinot Noir contiennent exclusivement des hydroxystilbènes glucosylés (Soleas *et al*., Clinical Biochemistry, vol. 30, Mars 1997).

Différentes études *in vitro* et *in vivo* ont mis en évidence les propriétés biologiques intéressantes des hydroxystilbènes, en particulier leurs propriétés anti-inflammatoires, anti-oxydantes et anti-mutagènes, et leur influence sur le métabolisme des lipides et sur l'aggrégation des plaquettes (Soleas *et al.*, 1997 ; Jang *et al*., Science, vol. 275, 10 Janvier 1997).

Ces propriétés ont été mises à profit dans la réalisation de compositions cosmétiques contenant ces composés. Il est décrit par exemple des compositions cosmétiques comprenant du resvératrol et leurs utilisations pour lutter contre les signes du vieillissement cutané, pour lisser la peau ou pour traiter les rides et les ridules (WO 99/04747, Unilever, N.V.). Il est aussi décrit un procédé d'obtention de dérivés esters de resvératrol et leurs utilisations dans des compositions cosmétiques en tant que précurseur de resvératrol (WO 99/03816, Caudalie).

Malgré ces propriétés intéressantes, les hydroxystilbènes présentent certains inconvénients dans le cadre notamment de leur utilisation dans le domaine cosmétique. Ainsi, ils peuvent s'oxyder sous l'action des enzymes de la flore cutanée et de ce fait perdre leurs propriétés intéressantes.

En outre, certains hydroxystilbènes ne sont solubles et stables que dans l'éthanol, qui ne peut être utilisé dans des formulations cosmétiques. Les hydroxystilbènes constituent de plus une matière première onéreuse.

Afin de mettre en oeuvre, principalement au niveau de la peau, les propriétés intéressantes des hydroxystilbènes, tout en surmontant certains inconvénients de ces principes actifs, les inventeurs se sont intéressés à certains de leurs dérivés. Ils ont mis en évidence que chez l'homme, des enzymes présentes au niveau de la peau ou du follicule pileux, et en particulier au niveau du stratum corneum, pouvaient convertir les dérivés glucosylés d'hydroxystilbènes en hydroxystilbènes et ce de façon à en tirer des avantages comparables, voire supérieurs, à ceux qui ont pu être décrits du fait de l'application topique, sur la peau, d'hydroxystilbènes.

Dans une première approche expérimentale, les inventeurs se sont intéressés au resvératrol glucosylé qui a l'avantage d'être plus stable et plus soluble que le resvératrol et est donc plus apte à être utilisé dans une composition cosmétique. Il est de plus naturellement présent dans certains végétaux et aisément extractible. Une étude a cependant montré que la forme glucosylée possède une activité antioxydante bien inférieure à la forme non glucosylée (Teguo *et al*., 1998).

Les inventeurs ont mis en évidence, qu'utilisés en tant que principes actifs dans des compositions cosmétiques, dermatologiques ou pharmaceutiques, appropriées pour l'application topique, les dérivés d'hydroxystilbènes peuvent par action de glucosidases endogènes de la peau, plus particulièrement du stratum corneum, permettre la libération *in vivo* de composés actifs ayant des propriétés avantageuses et un effet favorable au niveau de la microcirculation (par une meilleure oxygénation cellulaire), et notamment un effet anti-oxydant et/ou anti-inflammatoire *in vivo*.

L'invention porte donc sur une composition appropriée pour une application topique comprenant des hydroxystilbènes glucosylés de formule I générale suivante : 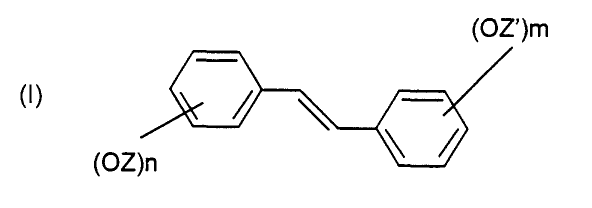 dans laquelle n est un nombre entier compris entre 1 et 5 inclusivement et m est un nombre entier compris entre 0 et 5 inclusivement, et Z et Z', identiques ou différents, représentent un atome d'hydrogène ou un radical glucosyle, étant entendu qu'au moins un Z ou Z' est un radical glucosyle. Ces composés peuvent être sous une forme cis ou trans.

Selon l'invention, le terme hydroxystilbène recouvre aussi bien les composés de formule I que leurs dérivés hydroxyalkylés.

Des dérivés d'hydroxystilbènes particulièrement avantageux pour la réalisation de compositions selon l'invention incluent par exemple le resvératrol glucosylé mis en oeuvre dans des conditions telles qu'il est transformé *in vivo* en resvératrol.

L'expression « resvératrol glucosylé » utilisée dans le cadre de la présente demande de brevet, comprend tous les composés dérivés du resvératrol de formule (II) suivante : 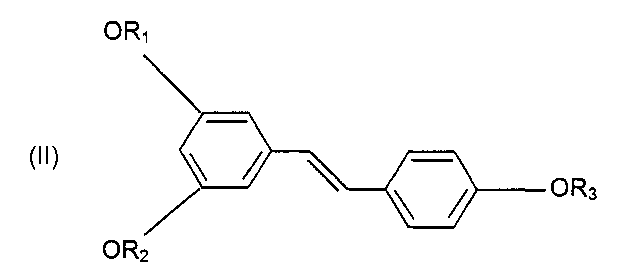 dans laquelle : R1, R2 et R3, identiques ou différents, représentent un groupement hydroxyle ou un groupement glucosyle, étant entendu qu'au moins R1 ou R2 ou R3 est un groupement glucosyle.

Des exemples d'hydroxystilbènes glucosylés sont :
- Les composés répondant à la formule ci-dessus et en particulier le 3,4'-dihydroxystilbène-5-O-béta-glucoside; le 3,5-dihydroxystilbène-4'-O-béta-glucoside ; le 4',5-dihydroxystilbène-3-O-béta-glucoside ; le 4'-hydroxystilbène-3,5-O-béta-di-glucoside ; le 5-hydroxystilbène-3,4'-O-béta-di-glucoside ; le 3-hydroxystilbène-4',5-O-béta-di-glucoside et le stilbène-3,4',5-O-béta-tri-glucoside,
- le 4'-méthoxy-3',5-stilbènediol-3-O-béta-glucoside,
- le 3,5,4'-trihydroxystilbène-2-O-béta-glucoside,
- le 3',4,5'-trihydroxystilbène-3-O-béta-glucoside,
- la pinosylvine glucoside et en particulier les composés suivants : 5-hydroxystilbène-3-O-béta-glucoside, 3-hydroxystilbène-5-O-béta-glucoside ou stilbène-3,5-O-béta-di-glucoside.

L'invention concerne les formes D ou L glucoside des hydroxystilbènes, ou un mélange racémique de ces formes. De préférence, l'invention concerne les formes D. Un composé très préférentiel de l'invention est le 4',5-dihydroxystilbène-3-O-béta-D-glucoside.

Dans un mode de réalisation particulier de l'invention, les compositions comprennent un mélange d'hydroxystilbènes glucosylés et avantageusement un mélange de composés appartenant à la famille du resvératrol glucosylé.

L'invention propose donc l'administration, par voie topique, d'hydroxystilbènes glucosylés et en particulier un des composés ou un mélange de composés identifiés spécifiquement ci-dessus, par exemple dans une application cosmétique ou pharmaceutique.

Par conséquent, l'invention fournit un procédé de libération progressive des hydroxystilbènes au niveau du stratum corneum par application topique d'une composition comprenant des hydroxystilbènes glucosylés et en particulier un des composés ou un mélange de composés identifiés spécifiquement ci-dessus.

Pour la réalisation de l'invention, les hydroxystilbènes glucosylés et en particulier, un des composés ou un mélange de composés identifiés spécifiquement ci-dessus, peuvent être extraits de végétaux ou de parties de végétaux. Ils peuvent être aussi synthétisés par voie chimique.

Lorsque, pour la préparation des compositions selon l'invention, les hydroxystilbènes glucosylés sont isolés de végétaux ou de matériel végétal le contenant, les plantes de familles suivantes peuvent être utilisées : Vitacées, Ombellifères, Myrtacées, Diptérocarpacées, Cypéracées, Gnétacées, Légumineuses, Graminées, Séricées, Haemodoracées, Musacées, polygonacées, Pinacées, Crupressacées, Césalpiniacées, Poacées et Solanacées. Plus particulièrement, ils sont isolés des tissus de *Vitis vinifera* ou de *Polygonum cuspidatum*, de préférence, ils sont isolés de la peau du raisin. Ils peuvent aussi être extraits de produits dérivés du raisin tels que le vin.

Les hydroxystilbènes glucosylés et en particulier un des composés ou un mélange de composés identifiés spécifiquement ci-dessus peuvent être extraits et purifiés par une procédure d'extraction de A.L. Waterhouse (Phytochemistry vol. 37 p.571 (1994)).

Une forme préférée d'hydroxystilbènes pour la réalisation de compositions selon l'invention est le resvératrol glucosylé. Une méthode simple d'obtention d'une fraction enrichie en resvératrol glucosylé, consiste en une extraction au méthanol et/ou à l'éthanol/eau. Par exemple, 100 g de *Polygonum cuspidatum* sont mélangés avec 800 ml d'eau et 200 ml d'éthanol, le mélange est agité vigoureusement pendant 12 heures à 4°C puis filtré. Ce filtrat peut être délipidé à l'éther de pétrole par exemple et repris ou non avec de l'eau puis évaporé.

Le resvératrol glucosylé peut aussi être extrait à partir de cultures *in vitro* de cellules de *Vitis vinifera*. Des méthodes d'extraction du resvératrol glucosylé ont par ailleurs été décrites par Téguo *et al*. (Téguo et al., 1998) ou dans la demande de brevet PCT WO 99/03816.

Selon l'invention, les hydroxystilbènes glucosylés et en particulier, un des composés ou un mélange de composés identifiés spécifiquement ci-dessus, représentent entre 0.01% et 10% du poids total de la composition, de manière préférée entre 0.1% et 5% du poids total de la composition.

Les compositions de l'invention se présentent sous forme de crème, de pommade, d'émulsion, de gel ou de lotion.

Un des avantages des compositions selon l'invention est la possibilité de moduler la cinétique de la réaction enzymatique conduisant à la libération des hydroxystilbènes à partir d'hydroxystilbènes glucosylés (bioconversion) notamment à partir d'un des composés ou un mélange de composés identifiés spécifiquement ci-dessus, et plus particulièrement à partir du resvératrol glucosylé.

Les compositions peuvent comprendre des activateurs ou inhibiteurs de glucosidases ou tout produit conduisant à une modulation de la cinétique de bioconversion. Des activateurs sont donc notamment ajoutés à la composition cosmétique ou pharmaceutique afin de stimuler l'activité de glucosidases endogènes. De tels activateurs sont par exemple le 1-O-méthyl-β-D-glucopyranoside. Ces activateurs représentent entre 0.01% et 10% du poids total de la composition, de manière préférée entre 0.1% et 5% du poids total de la composition.

Selon un mode préféré de réalisation de l'invention, la composition selon l'invention a un pH proche de celui de la peau et de préférence allant de 4 à 7. Il s'ensuit une grande compatibilité et tolérance de la composition selon l'invention vis-à-vis de la peau.

La composition de l'invention est destinée à une application topique et comprend de manière appropriée un milieu physiologiquement acceptable. Par milieu physiologiquement acceptable, on entend un milieu compatible avec la peau, les muqueuses (y compris l'intérieur des paupières et les lèvres), les ongles et/ou les fibres kératiniques (cheveux et cils).

En outre, de façon connue, les compositions de l'invention peuvent contenir des adjuvants habituels dans les domaines cosmétique et/ou pharmaceutique, tels que les actifs hydrophiles ou lipophiles, les conservateurs, les antioxydants, les parfums, les charges, les matières colorantes (pigments ou colorants), les filtres solaires, les solvants et encore les vésicules lipidiques. Ces adjuvants sont utilisés dans les proportions habituelles dans le domaine cosmétique ou dermatologique, et par exemple de 0,01 à 20% du poids total de la composition, et ils sont, selon leur nature, introduits dans une phase aqueuse ou dans une phase huileuse de la composition, ou encore dans des vésicules.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

Le filtre solaire est de préférence choisi parmi les filtres organiques et/ou les filtres minéraux.

Comme filtres organiques, on peut notamment citer les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665 ou encore les filtres organiques décrits dans la demande de brevet EP-A 0 487 404.

Comme filtres minéraux, on peut notamment citer des pigments ou bien encore des nanopigments (taille moyenne des particules primaires : généralement entre 5nm et 10 nm, de préférence entre 10 et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particlier décrits dans les demandes de brevets EP-A-0 518 772 et EP-A-0 518 773.

Comme exemples de filtres solaires complémentaires actifs dans l'UV-A et/ou l'UV-B, on peut citer :
- l'acide p-aminobenzoïque,
- le p-aminobenzoate oxyéthyléné (25 mol),
- le p-diméthylaminobenzoate de 2-éthylhexyle,
- le p-aminobenzoate d'éthyle N-oxypropyléné,
- le p-aminobenzoate de glycérol,
- le salicylate d'homomenthyle,
- le salicylate de 2-éthylhexyle,
- le salicylate de triéthanolamine,
- le salicylate de 4-isopropylbenzyle,
- le 4-ter-butyl-4'-méthoxy-dibenzoylméthane (PARSOL 1789 de GIVAUDAN ROURE)
- le p-méthoxycinnamate de 2-éthylhexyle (PARSOL MCX de GIVAUDAN ROURE)
- le 4-isopropyl-dibenzoylméthane (EUSOLEX 8020 de MERCK),
- l'anthranilate de menthyle,
- le 2-éthylhexyl-2-cyano-3,3'-diphénylacrylate, (UVINUL N539 de BASF),
- l'éthyl-2-cyano-3,3'-diphénylacrylate,
- l'acide 2-phényl benzimidazole 5-sulfonique et ses sels,
- le 3-(4'-triméthylammonium)-benzylidèn-bornan-2-on-méthylsulfate,
- le 2-hydroxy-4-méthoxybenzophénone (UVINUL MS 40 de BASF),
- le 2-hydroxy-4-méthoxybenzophénone-5-sulfonate (UVINUL MS 40 de BASF),
- le 2,4-dihydroxybenzophénone (UVINUL 400 de BASF),
- le 2,2',4,4'-tétrahydroxybenzophénone (UVINUL D 50 de BASF),
- le 2,2'-dihydroxy-4,4'-diméthoxybenzophénone (HELISORB II de NORQUAY),
- le 2-hydroxy-4-n-octoxybenzophénone,
- le 2-hydroxy-4-méthoxy-4'-méthylbenzophénone,
- l'acide α-(2-oxoborn-3-ylidène)-tolyl-4-sulfonique et ses sels,
- le 3-(4'-sulfo)benzylidèn-bornan-2-one et ses sels,
- le 3-(4'-méthylbenzylidène)-d,l-camphre,
- le 3-benzylidène-d,l-camphre,
- l'acide benzène 1,4-di(3-méthylidène-10-camphosulfonique) et ses sels (MEXORYL SX de CHIMEX),
- l'acide urocanique,
- le 2,4-6-tris-[p-(2'-éthylhexyl-1'-oxycarbonyl)anilino]-1,3,5-triazine,
- le 2-[p-(tertiobutylamido)anilino]-4,6-bis [p-(2'-éthylhexyl-1'-oxycarbonyl)anilino]-1,3,5-triazine,
- le 2,4-bis{[4-2-éthyl-hexyloxyl]-2-hydroxyl-phenyl }-6-(4-méthoxyphenyl)-1,3,5-triazine,
- le polymère de N-(2 et 4)-[2-oxoborn-3-ylidèn)méthyl)benzyl]-acrylamide,
- l'acide 4,4-bis-benzimidazolyl-phénylèn-3,3',5,5'-tétrasulfonique et ses sels,
- le 2,2'-méthylèn-bis-[6-(2H-benzotriazol-2-yl)-4-(1,1,3,3-tétraméthylbutyl) phénol],
- les polyorganosiloxanes à fonction malonate.

Comme solvant que l'on peut utiliser dans la composition de l'invention, on peut citer par exemple l'octyldodécanol.

Selon la fluidité de la composition que l'on souhaite obtenir, on peut y ajouter un ou plusieurs gélifiants comme les argiles, les gommes polysaccharides et leurs dérivés (gomme de xanthane, carboxyméthyl cellulose, hydroxypropyl guar), les polymères carboxyvinyliques ou carbomers, les polyacrylamides tels que celui commercialisé sous la dénomination SEPIGEL 305 par la société SEPPIC et les polymères d'acide acrylamidométhylpropanesulfonique au moins partiellement réticulés tels que le produit commercialisé sous la dénomination HOSTACERIN AMPS par la société HOECHST. Ces gélifiants sont généralement utilisés à des concentrations allant de 0,1 à 10% de préférence 0,1 à 5% et mieux de 0,1 à 3% du poids total de la composition.

L'invention a encore pour objet un procédé de traitement cosmétique pour le contrôle de la pigmentation de la peau, pour lutter contre les signes du vieillissement cutané et du follicule pileux, pour améliorer l'éclat du teint, pour lisser la peau du visage, pour traiter ou prévenir les rides et les ridules de la peau ou pour stimuler le processus de renouvellement épidermique, consistant à appliquer sur la peau une composition telle que définie ci-dessus.

Elle porte enfin sur des utilisations des hydroxystilbènes glucosylés et en particulier un des composés ou un mélange de composés identifiés spécifiquement ci-dessus comme précurseur des hydroxystilbènes par l'application topique des compositions cosmétiques ou pharmaceutiques le ou les comprenant.

Les exemples qui suivent présentent une illustration de la libération de resvératrol à partir de resvératrol glucosylé en présence d'extraits solubles du stratum corneum ainsi qu'une illustration d'un mode de réalisation des compositions selon l'invention, sans être limitatifs.

### Exemple 1 :

Cet exemple montre qu'il est possible de libérer le resvératrol à partir de resvératrol glucosylé en présence d'extrait de stratum corneum ou d'un homogénat de follicule pileux. Il montre de plus que l'addition d'un activateur des glucosidases permet d'augmenter de manière siginificative la réaction d'hydrolyse.

Un resvératrol glucosylé (4',5-dihydroxystilbène-3-O-béta-D-glucoside) (APIN Chemicals N17555p, 29 D. Milton Park, Abingtdon, Oxon, United Kingdom) est incubé en présence d'un extrait de stratum corneum ou d'un homogénat de follicule pileux prélevé par arrachage. Dans des conditions expérimentales précises, l'affinité du resvératrol pour la tyrosinase est supérieure au resvératrol glucosylé. Cette propriété est avantageusement utilisée pour mesurer le resvératrol libéré, déterminé à 255 nm, en présence de tyrosinase. Cette méthode est adaptée de la méthode décrite par A.A. Calderon : A spectrophotometric assay for quantitative analysis of the oxidation of 4-hydroxystilbene by peroxidase-H₂O₂ systems; *J. of Biochem*. *and Biophys.* (1990) Methods **20** : 171-180.

### 1. Matériels et solutions utilisées

- 4',5-dihydroxystilbène-3-O-béta-D-glucoside : 1 mM dans l'éthanol.
- Resvératrol (SIGMA R501 0) : 1 mM dans l'éthanol.
- Tyrosinase (SIGMA T7755) 600 unités / ml PBS
- Activateur: 1-0-méthyl-β-D-glucopyranoside (Biosynth: Biochemica et synthetica) 1 mM dans du PBS.
- Tampon PBS pH 7,2

Un extrait de cellules du stratum comeum est prélevé par grattage en présence de PBS (10 ml sur une zone de 20 cm² environ), puis filtré sur un filtre 0.22 µm pour obtenir une solution de stratum comeum.

5 follicules sont prélevés chez l'homme par arrachage et homogénéisés dans 100 µL de PBS pour obtenir un homogénat de follicules pileux.

400 µL de solution de stratum corneum sont incubés en présence de resvératrol glucoside (50 µl) avec et sans activateur (25 µl).

20 µl d'homogénat de follicules pileux sont incubés en présence de resvératrol glucoside (50 µl) avec et sans activateur (10 µl).

Le volume est ajusté à 500 µl avec du PBS et l'incubation est de 5 heures à 37 °C à 25°C.

Un aliquote de 200 µl est prélevé et 10 µl de tyrosinase sont ajoutés.

Deux mesures indépendantes sont effectuées par évaluation de la densité optique à 255 nm pendant 10 minutes et calcul de la pente (60 à 300 secondes).

Dans les mêmes conditions une gamme étalon est réalisée avec le resvératrol (1 ; 2,5 ; 5 ; 7,5 et 10 µl) dans un volume ajusté à 200 µl.

### 2. Résultats

Le tableau 1 suivant présente les résultats des tests *in vitro* avec le resvératrol glucosylé seul ou à titre d'exemple, en présence de l'activateur de glucosidase (1-0-méthyl-β-D-glucopyranoside) à deux concentrations différentes.

**Tableau 1 :**

| **STRATUM CORNEUM** | |
|---|---|
| Resvératrol libéré : | |
| • sans activateur | 2,85 nmoles |
| • activateur 0,5 mM | 5,65 nmoles (+ 98 %) |
| • activateur 0,1 mM | 4,00 nmoles (+ 40 %) |

| **HOMOGENAT FOLLICULES PILEUX** | |
|---|---|
| Resvératrol libéré : | |
| • sans activateur | 6,15 nmoles |
| • activateur 0,5 mM | 7,15 nmoles (+ 16%) |
| • activateur 0,1 mM | 6,70 nmoles (+ 9%) |

Les résultats obtenus sur les tests *in vitro* montrent une libération significative du resvératrol à partir de resvératrol glucosylé. Cette libération peut de plus être augmentée en présence d'activateurs de glucosidases. Il a par ailleurs été montré par des tests *in vitro* une action du resvératrol à des concentrations relativement faibles, inférieures à 10 µm. Une libération limitée mais continue dans le temps du resvératrol à partir de resvératrol glucosylé est donc particulièrement adaptée pour des applications cosmétiques.

Les pourcentages entre parenthèses indiquent le pourcentage d'augmentation du nombre de molécules de resvératrol libéré par rapport au contrôle en absence d'activateurs.

### Exemple 2 : Exemple de formulation d'une composition cosmétique selon l'invention

Une composition cosmétique selon l'invention est formulée de la manière suivante :

### Crème traitante

Alcool cétylique 1.05%
Stéarate de PEG 20 (Myrj 49 vendu par la société ICI) 2%
Cyclométhicone 6%
4',5-dihydroxystilbène-3-O-béta-mono-D-glucoside 0.5%
1-O-méthyl-béta-D-glucopyranoside 0.3%
Carbomer 0.6%
Glycérine 3%
Triéthanolamine 1%
conservateurs 0.5%
eau déminéralisée qsp 100%

## Revendications

1. Composition appropriée pour une application topique, comprenant des hydroxystilbènes glucosylés de formule I générale : 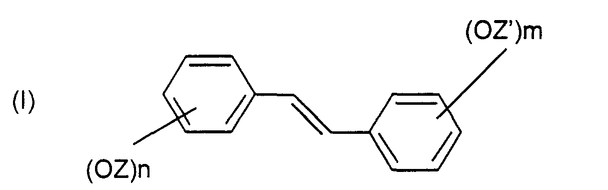 dans laquelle n est un nombre entier compris entre 1 et 5 inclusivement et m est un nombre entier compris entre 0 et 5 inclusivement, et Z et Z', identiques ou différents, représentent un atome d'hydrogène ou un radical glucosyle, étant entendu qu'au moins un Z ou Z' est un radical glucosyle.

2. Composition selon la revendication 1 dans laquelle un hydroxystilbène glucosylé est le composé « resvératrol glucosyle » de formule II générale suivante : 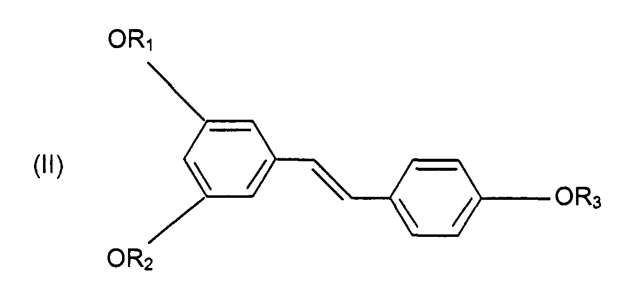 dans laquelle : R1, R2 et R3, identiques ou différents, représentent un groupement hydroxyle ou un groupement glucosyle, étant entendu qu'au moins R1 ou R2 ou R3 est un groupement glucosyle.

3. Composition selon la revendication 1 dans laquelle un hydroxystilbène glucosylé ou un mélange de composés est choisi parmi le groupe des composés suivants :
- Le 3,4'-dihydroxystilbène-5-O-béta-glucoside,
- le 3,5-dihydroxystilbène-4'-O-béta-glucoside,
- le 4',5-dihydroxystilbène-3-O-béta-glucoside,
- le 4'-hydroxystilbène-3,5-O-béta-di-glucoside,
- le 5-hydroxystilbène-3,4'-O-béta-di-glucoside,
- le 3-hydroxystilbène-4',5-0-béta-glucoside,
- le stilbène-3,4',5-O-béta-tri-glucoside,
- le 4'-méthoxy-3',5-stilbènediol-3-O-béta-glucoside,
- le 3,5,4'-trihydroxystilbène-2-O-béta-glucoside,
- le 3',4,5'-trihydroxystilbène-3-O-béta-glucoside,
- le 5-hydroxystilbène-3-O-béta-glucoside ;
- le 3-hydroxystilbène-5-O-béta-glucoside ;
- le stilbène-3,5-O-béta-di-glucoside.

4. Composition selon la revendication 2 dans laquelle un resvératrol glucosylé est le 4',5-dihydroxystilbène-3-O-béta-D-glucoside.

5. Composition selon l'une quelconque des revendications 2 ou 4 **caractérisée en ce que** le resvératrol glucosylé est isolé de cellules de *vitis vinifera* cultivées *in vitro*.

6. Composition selon l'une quelconque des revendications 1 à 5 **caractérisée en ce qu'**elle comprend en plus un activateur de glucosidases.

7. Composition selon la revendication 6 **caractérisée en ce qu'**un activateur des glucosidases est le 1-O-méthyl-β-D-glucopyranoside.

8. Composition selon l'une quelconque des revendications 6 ou 7 **caractérisée en ce qu'**elle comprend entre 0,01% et 10% d'activateurs de glucosidases, de préférence entre 0,1% et 5%.

9. Composition selon l'une quelconque des revendications 1 à 5 **caractérisée en ce qu'**elle comprend en plus l'un des inhibiteurs des glucosidases.

10. Composition selon l'une quelconque des revendications 1 à 9 **caractérisée en ce que** l'hydroxystilbène glucosylé est extrait de végétaux.

11. Composition selon la revendication 10 **caractérisée en ce que** le resvératrol glucosylé est extrait des tissus de *vitis vinifera* ou de *Polygonum cuspidatum,* en particulier de la peau du raisin.

12. Composition selon l'une quelconque des revendications 1 à 9 **caractérisée en ce que** l'hydroxystilbène glucosylé est extrait du vin.

13. Composition selon l'une quelconque des revendications 1 à 12 **caractérisée en ce qu'**elle est formulée à un pH compris entre 4 et 7.

14. Composition selon l'une quelconque des revendications 1 à 13 **caractérisée en ce qu'**elle comprend entre 0,01% et 10% d'hydroxystilbènes glucosylés, de préférence entre 0,1% et 5%.

15. Procédé cosmétique de libération d'hydroxystilbènes au niveau du stratum corneum par application sur la peau d'une composition **caractérisée** selon l'une quelconque des revendications 1 à 14.

16. Utilisation des hydroxystilbènes glucosylés comme précurseur d'hydroxystilbènes pour le contrôle de la pigmentation de la peau, pour lutter contre les signes du vieillissement cutané et du follicule pileux, pour améliorer l'éclat du teint, pour lisser la peau du visage, pour traiter ou prévenir les rides et les ridules de la peau, pour stimuler le processus de renouvellement épidermique par application sur la peau d'une composition appropriée pour l'application topique selon l'une quelconque des revendications 1 à 14.

17. Utilisation des hydroxystilbènes glucosylés comme précurseur d'hydroxystilbènes selon le procédé de la revendication 15.

18. Utilisation des hydroxystilbènes glucosylés comme précurseur d'hydroxystilbènes dans une composition à application topique.
